# EUROPÄISCHE PATENTSCHRIFT

(11) **EP 2 217 574 B1**
(45) Veröffentlichungstag und Bekanntmachung des Hinweises auf die Patenterteilung: **10.04.2013**
(21) Anmeldenummer: 08848332.6
(22) Anmeldetag: 31.10.2008
(51) Int. Cl.: C07D 233/58, C07C 277/00

(54) **VERFAHREN ZUR HERSTELLUNG IONISCHER FLÜSSIGKEITEN DURCH ANIONENAUSTAUSCH**
METHOD FOR PRODUCING IONIC LIQUIDS BY ANION EXCHANGE
PROCÉDÉ POUR LA PRODUCTION DE LIQUIDES IONIQUES PAR ÉCHANGE D'ANIONS

(30) Priorität: 08.11.2007 DE 102007053630
(43) Veröffentlichungstag der Anmeldung: 18.08.2010
(73) Patentinhaber: BASF SE, 67056 Ludwigshafen (DE)
(72) Erfinder: MASSONNE, Klemens, 67098 Bad Dürkheim (DE); SIEMER, Michael, 68159 Mannheim (DE); MORMANN, Werner, 57076 Siegen (DE); LENG, Wei, 49448 Lemförde (DE)
(86) Internationale Anmeldenummer: PCT/EP2008/064794
(87) Internationale Veröffentlichungsnummer: WO 2009/059934

(56) Entgegenhaltungen:
- WO-A-2006/027070

## Beschreibung

Die Erfindung betrifft ein Verfahren zur Herstellung von Salzen der Formel I

(B⁺)ₙ × A^{y-}

wobei
B für ein Kation, welches ein heterocyclisches Ringsystem mit mindestens einem Stickstoffatom enthält,
A für ein Anion und
n für eine ganze Zahl von 1 bis 3 steht
x und y jeweils für eine ganze Zahl von 1 bis 3 stehen und das Produkt aus x und y gleich n ist,
durch Umsetzung von Salzen der Formel II

(B⁺)ₙ × C^{y-}

worin B und n, x und y die obige Bedeutung haben und C für eine von A verschiedene Verbindung mit einer oder mehreren Carboxylatgruppen (kurz Carboxylate genannt) steht,
mit dem Ammoniumsalz des Anions A oder mit der Protonensäure des Anion A in Gegenwart von Ammoniak.

Salze mit einem Schmelzpunkt kleiner 200°C, insbesondere mit einem Schmelzpunkt kleiner 100°C, werden als ionische Flüssigkeiten bezeichnet. Von besonderem Interesse sind ionische Flüssigkeiten, welche bereits bei Raumtemperatur flüssig sind.

Für ionische Flüssigkeiten gibt es verschiedene Herstellungsverfahren. Ionische Flüssigkeiten mit Ammoniumkationen und Carboxylatanionen können z.B. nach der in WO 2005/021484 (Carbonatmethode) oder in WO 91/14678 (Arduengo-Verfahren) beschriebenen Methoden hergestellt werden. Ionische Flüssigkeiten mit anderen Anionen als Carboxylatanionen sind durch anschließenden Austausch des Anions erhältlich.

WO 2006/027070 beschreibt einen derartigen Anionenaustausch unter Verwendung einer Protonensäure mit einem pKa Wert ≤ 14. Nach dem beschriebenen Verfahren ist ein vollständiger Anionenaustausch nicht oder nur durch aufwendige Verfahrensmaßnahmen, z.B. durch mehrmaliges Destillieren, zu erreichen.

Aufgabe der vorliegenden Erfindung ist daher ein einfaches und effektives Verfahren zur Herstellung von Salzen, insbesondere ionischen Flüssigkeiten, durch Anionenaustausch.

Demgemäß wurde das eingangs definierte Verfahren gefunden.

Zum Kation B der Salze der Formel I

Beim erfindungsgemäßen verfahren werden Salzen der obigen Formel I hergestellt.

Kationen B enthalten ein heterocyclisches Ringsystem mit mindestens einem Stickstoffatom.

Besonders bevorzugte Kationen B enthalten ein aromatisches, heterocyclisches Ringsystem mit mindestens einem Stickstoffatom und einer delokalisierten positiven Ladung.

Geeignete Kationen sind insbesondere Derivate des Imidazoliums, des Imidazoliniums, des Pyrazoliums, des Pyrazoliniums und des Pyridiniums.

Kationen B enthalten ein heterocyclisches Ringsystem mit mindestens einem Stickstoffatom.

Besonders bevorzugte Kationen B enthalten ein aromatisches, heterocyclisches Ringsystem mit mindestens einem Stickstoffatom und einer delokalisierten positiven Ladung.

Geeignete Kationen sind insbesondere Derivate des Imidazoliums, des Imidazoliniums, des Pyrazoliums, des Pyrazoliniums und des Pyridiniums.

Geeignete Imidazolinium-Kationen besitzen bevorzugt die allgemeine Formel (VI) 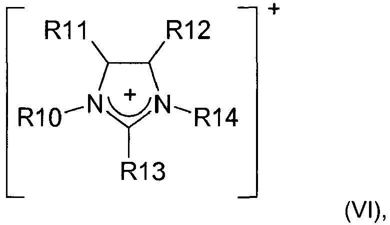 in der
die Reste R11 bis R14 unabhängig voneinander eine Sulfo-Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten und die Reste R11 bis R13 zusätzlich noch unabhängig voneinander Wasserstoff, Halogen oder eine funktionelle Gruppe bedeuten und der Rest R14 zusätzlich auch für Wasserstoff stehen kann; oder
zwei benachbarte Reste R11 zusammen mit R12; oder R12 zusammen mit R14; oder R14 zusammen mit R13 zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste wie zuvor definiert sind;
und der Rest R10 einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeutet.

Geeignete Pyrazolium-Kationen besitzen bevorzugt die allgemeine Formel (VII) 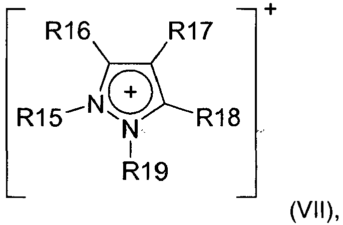 in der
die Reste R16 bis R19 unabhängig voneinander eine Sulfo-Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten und die Reste R16 bis R18 zusätzlich noch unabhängig voneinander Wasserstoff, Halogen oder eine funktionelle Gruppe bedeuten und der Rest R19 zusätzlich auch für Wasserstoff stehen kann; oder
zwei benachbarte Reste R16 zusammen mit R17; oder R17 zusammen mit R18; oder R18 zusammen mit R19 zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste wie zuvor definiert sind;
und der Rest R15 einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeutet.

Geeignete Pyrazolinium-Kationen besitzen bevorzugt die allgemeine Formel (VIII) 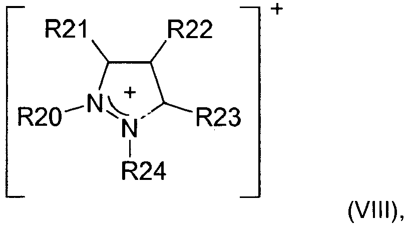 in der
die Reste R21 bis R24 unabhängig voneinander eine Sulfo-Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten und die Reste R21 bis R23 zusätzlich noch unabhängig voneinander Wasserstoff, Halogen oder eine funktionelle Gruppe bedeuten und der Rest R24 zusätzlich auch für Wasserstoff stehen kann; oder
zwei benachbarte Reste R21 zusammen mit R22; oder R22 zusammen mit R24; oder R24 zusammen mit R23 zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste wie zuvor definiert sind;
und der Rest R20 einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeutet.

Geeignete Pyridinium-Kationen besitzen bevorzugt die allgemeine Formel (IX) 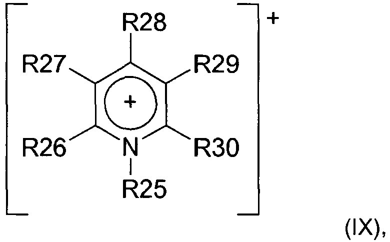 in der
die Reste R26 bis R30 unabhängig voneinander Wasserstoff, Halogen, eine funktionelle Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten; oder
zwei benachbarte Reste R26 zusammen mit R27; oder R27 zusammen mit R28; oder R28 zusammen mit R29 oder R29 zusammen mit R30 zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste wie zuvor definiert sind;
und der Rest R25 einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeutet.

Zu dem Anion A der Salze der Formel I

Bei dem erfindungsgemäßen Verfahren wird ein Carboxylat - Anion (siehe Formel II) gegen ein anderes Anion ausgetauscht. Soweit A in Formel I für ein Carboxylat steht, sollte es sich um ein anderes Carboxylat als in Formel II handeln.

Das Anion A kann ein-, zwei- oder dreiwertig sein. Vorzugsweise ist A einwertig (y = 1). Entsprechen ist dann auch x = 1 und n = 1.

Insbesondere handelt es sich bei dem Anion um
Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat, Vinylphosphonat, Dicyanamid, Bis(penta-fluoroethyl)phosphinat, Tris(pentafluoroethyl)trifluorophosphat, Tris(heptafluoropropyl)trifluorophosphat, Bis[oxalato(2-)]borat, Bis[salicylato(2-)]borat, Bis[1,2-benzol-diolato(2-)-O,O']borat, Tetracyanoborat, Tetracarbonylcobaltat;
tetrasubstituiertes Borat, insbesondere der allgemeinen Formel (Va) [BR^{a}R^{b}R^{c}R^{d}]-, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfonat, insbesondere der allgemeinen Formel (Vb) [Re-SO₃]-, wobei Re für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Carboxylat, insbesondere der allgemeinen Formel (Vc) [R^{f}-COO]-, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen (wobei es sich auch um weitere Carboxylatgruppen handeln kann) oder Halogen substituiert sein kann, steht;
(Fluoralkyl)fluorphosphat, insbesondere der allgemeinen Formel (Vd) [PFₓ(C_{y}F_{2y}+_{1-z}H_{z})₆₋ₓ]-, wobei 1 ≤ x ≤ 6, 1 ≤ y ≤ 8 und 0 ≤ z ≤ 2y+1;
Imid, insbesondere der allgemeinen Formeln (Ve) [R^{g}-SO₂-N-SO₂-R^{h}]-, (Vf) [Rⁱ-SO₂-N-CO-R^{j}]⁻ oder (IVg) [R^{k}-CO-N-CO-R^{l}]⁻, wobei R^{g} bis R^{l} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;

Methid, insbesondere der allgemeinen Formel (Vh) 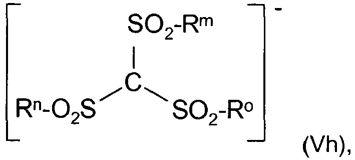 wobei R^{m} bis R^{o} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfat, insbesondere der allgemeinen Formel (Vi) [R^{p}O-SO₃]-, wobei R^{p} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht oder
Halometallat, insbesondere der allgemeinen Formel (Vj) [M_{q}Halᵣ]^{s-}, wobei M für ein Metall und Hal für Fluor, Chlor, Brom oder Iod steht, q und r ganze positive Zahlen sind und die Stöchiometrie des Komplexes angeben und s eine ganze positive Zahl ist und die Ladung des Komplexes angibt; ist.

Als Heteroatome in den vorstehenden Formeln kommen prinzipiell alle Heteroatome in Frage, welche in der Lage sind, formell eine -CH₂-, eine -CH=, eine C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen insbesondere-O-, -S-, -SO-, -SO₂-, -NR-, -N=, -PR-, -PR₂ und -SiR₂- genannt, wobei es sich bei den Resten R um den verbleibenden Teil des Kohlenstoff enthaltenden Rests handelt.

Als funktionelle Gruppen in den vorstehenden Formeln kommen prinzipiell alle funktionellen Gruppen in Frage, welche an ein Kohlenstoffatom oder ein Heteroatom gebunden sein können. Als geeignete Beispiele seien -OH (Hydroxy), =O (insbesondere als Carbonylgruppe), -NH₂ (Amino), =NH (Imino), -COOH (Carboxy), -CONH₂ (Carboxamid) und -CN (Cyano) genannt. Fuktionelle Gruppen und Heteroatome können auch direkt benachbart sein, so dass auch Kombinationen aus mehreren benachbarten Atomen, wie etwa -O- (Ether), -S- (Thioether), -COO- (Ester), -CONH- (sekundäres Amid) oder -CONR- (tertiäres Amid), mit umfasst sind,

Als Halogene seien Fluor, Chlor, Brom und Iod genannt.

Als Kohlenstoff enthaltende organische, gesättigte oder ungesättigte, acyclische oder cyclische, aliphatische, aromatische oder araliphatische Reste mit 1 bis 30 Kohlenstoffatomen stehen die Reste R^{a} bis R^{d} beim tetrasubstituiertes Borat (Va), der Rest Re beim organischen Sulfonat (Vb), der Rest R^{f} beim Carboxylat (Vc), die Reste R^{g} bis R^{l} bei den Imiden (Ve), (Vf) und (Vg), die Reste R^{m} bis R^{o} beim Methid (Vh) und der Rest R^{p} beim organischen Sulfat (Vi) unabhängig voneinander bevorzugt für
- C₁- bis C₃₀-Alkyl und deren aryl-, heteroaryl-, cycloalkyl-, halogen-, hydroxy-, amino-, carboxy-, formyl-, -O-, -CO-, -CO-O- oder -CO-N< substituierte Komponenten, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl, Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl, Methoxy, Ethoxy, Formyl, Acetyl oder CₙF₂₍ₙ₋a)_{+(1-b)}H2_{a+b} mit n ≤30, 0 ≤ a ≤ n und b = 0 oder 1 (beispielsweise CF₃, C₂F₅, CH₂CH₂-C₍ₙ₋₂₎F₂₍ₙ₋₂₎₊₁, C₆F₁₃, C₈F₁₇, C₁₀F₂₁, C₁₂F₂₅);
- C₃- bis C₁₂-Cycloalkyl und deren aryl-, heteroaryl-, cycloalkyl-, halogen-, hydroxy-, amino-, carboxy-, formyl-, -O-, -CO- oder -CO-O-substituierte Komponenten, wie beispielsweise Cyclopentyl, 2-Methyl-1-cyclopentyl, 3-Methyl-1-cyclopentyl, Cyclohexyl, 2-Methyl-1-cyclohexyl, 3-Methyl-1-cyclohexyl, 4-Methyl-1-cyclohexyl oder CₙF_{2(n-a)-(1-b)}H_{2a-b} mit n ≤30, 0 ≤ a ≤ n und b = 0 oder 1;
- C₂- bis C₃₀-Alkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 2-Propenyl, 3-Butenyl, cis-2-Butenyl, trans-2-Butenyl oder CₙF_{2(n-a)-(1-b)}H_{2a-b} mit n ≤30, 0 ≤ au n und b = 0 oder 1;
- C₃- bis C₁₂-Cycloalkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 3-Cyclopentenyl, 2-Cyclohexenyl, 3-Cyclohexenyl, 2,5-Cyclohexadienyl oder CₙF_{2(n-a)-3(1-b)}H_{2a-3b} mit n ≤ 30, 0 ≤ a ≤ n und b = 0 oder 1; und
- Aryl oder Heteroaryl mit 2 bis 30 Kohlenstoffatomen und deren alkyl-, aryl-, heteroaryl-, cycloalkyl-, halogen-, hydroxy-, amino-, carboxy-, formyl-, -O-, -CO- oder -CO-O-substituierte Komponenten, wie beispielsweise Phenyl, 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenylphenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl oder C₆F₍₅₋ₐ₎Hₐ mit 0 ≤ a ≤ 5.

Handelt es sich beim Anion um ein tetrasubstituiertes Borat (Va) [BR^{a}R^{b}R^{c}R^{d}]-, so sind bei diesem bevorzugt alle vier Reste R^{a} bis R^{d} identisch, wobei diese bevorzugt für Fluor, Trifluormethyl, Pentafluorethyl, Phenyl, 3,5-Bis(trifluormethyl)phenyl oder cyanid (CN) stehen. Besonders bevorzugte tetrasubstituierte Borate (Va) sind Tetrafluoroborat, Tetraphenylborat und Tetra[3,5-bis(trifluormethyl)phenyl]borat.

Handelt es sich beim Anion um ein organisches Sulfonat (Vb) [Rₑ-SO₃]-, so steht der Rest Re bevorzugt für Methyl, Trifluormethyl, Pentafluorethyl, p-Tolyl oder C₉F₁₉. Besonders bevorzugte organische Sulfonate (Vb) sind Trifluormethansulfonat (Triflat), Methansulfonat, p-Tolylsulfonat, Nonadecafluorononansulfonat (Nonaflat), Dimethylenglykolmonomethyl-ethersulfat und Octylsulfat.

Handelt es sich beim Anion um ein Carboxylat (Vc) [R^{f}-COO]-, so steht der Rest R^{f} bevorzugt für Wasserstoff, Trifluormethyl, Pentafluorethyl, Phenyl, Hydroxy-phenylmethyl, Trichlormethyl, Dichlormethyl, Chlormethyl, Trifluormethyl, Difluormethyl, Fluormethyl, Ethenyl (Vinyl), 2-Propenyl, -OOC-(CH₂)ₙ mit n gleich 0, 1 oder 2, R"-OOC-(CH₂)ₙ mit R" gleich H oder C1 bis C8 Alkyl; CH=CH-COO-, CH=CH-COO-R" mit R" gleich H oder C1 bis C8 Alkyl, cis-8-Heptadecenyl,
CH₂-C(OH)(COOH)-CH₂₋COO- oder unverzweigtes oder verzweigtes C₁- bis C₁₈-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Heptadecyl. Besonders bevorzugte Carboxylate (Vc) sind Formiat, Acetat, Propionat, Butyrat, Valeriat, Benzoat, Mandelat, Trichloracetat, Dichloracetat, Chloracetat, Trifluoracetat, Difluoracetat, Fluoracetat.

Handelt es sich beim Anion um ein (Fluoralkyl)fluorphosphat (Vd) [PFₓ(C_{y}F_{2y+1-z}H_{z})₆₋ₓ]⁻, so ist z bevorzugt 0. Besonders bevorzugt sind (Fluoralkyl)fluorphosphate (Vd), bei. denen z = 0, x = 3 und 1 ≤ y ≤4, konkret [PF₃(CF₃)₃]⁻, [PF₃(C₂F₅)₃]⁻, [PF₃(C₃F₇)₃]⁻ und [PF₃(C₄F₇)₃]⁻.

Handelt es sich beim Anion um ein Imid (Ve) [R^{g}-SO₂-N-SO₂-R^{h}]⁻, (Vf)
[Rⁱ-SO₂-N-CO-R^{j}]⁻ oder (Vg) [R^{k}-CO-N-CO-R^{l}]⁻, so stehen die Reste R^{g} bis R^{l} unabhängig voneinander bevorzugt für Trifluormethyl, Pentafluorethyl, Phenyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Imide (Ve), (Vf) und (Vg) sind [F₃C-SO₂-N-SO₂-CF₃]⁻ (Bis(trifluoromethylsulfonyl)imid), [F₅C₂-SO₂-N-SO₂-C₂F₅]⁻ (Bis(pentafluoroethylsulfonyl)imid),
[F₃C-SO₂-N-CO-CF₃]⁻, [F₃C-CO-N-CO-CF₃]⁻ und jene, in denen die Reste R^{g} bis R^{l} unabhängig voneinander für Methyl, Ethyl, Propyl, Butyl, Phenyl, Trichlormethyl, Dichlormethyl, Chlormethyl, Trifluormethyl, Difluormethyl oder Fluormethyl stehen.

Handelt es sich beim Anion um ein Methid (Vh) 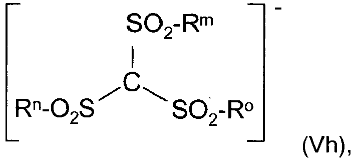 so stehen die Reste R^{m} bis R^{o} unabhängig voneinander bevorzugt für Trifluormethyl, Pentafluorethyl, Phenyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Methide (Vh) sind [(F₃C-SO₂)₃C]⁻ (Tris(trifluoro-methylsulfonyl)methid), [(F₅C₂-SO₂)₃C]⁻ (Bis(pentafluoroethylsulfonyl)methid) und jene, in denen die Reste R^{m} bis R^{o} unabhängig voneinander für Methyl, Ethyl, Propyl, Butyl, Phenyl, Trichlormethyl, Dichlormethyl, Chlormethyl, Trifluormethyl, Difluormethyl oder Fluormethyl stehen.

Handelt es sich beim Anion um ein organisches Sulfat (Vi) [R^{p}O-SO₃]⁻, so steht der Rest R^{p} bevorzugt für einen verzweigten oder unverzweigten C₁- bis C₃₀-Alklylrest. Besonders bevorzugte organische Sulfate (Vi) sind Methylsulfat, Ethylsulfat, Propylsulfat, Butylsulfat, Pentylsulfat, Hexylsulfat, Heptylsulfat oder Octylsulfat.

Handelt es sich beim Anion um ein Halometallat (Vj) [M_{q}Halᵣ]^{s-}, so steht M bevorzugt für Aluminium, Zink, Eisen, Cobald, Antimon oder Zinn. Hal steht bevorzugt für Chlor oder Brom und ganz besonders bevorzugt für Chlor. q ist bevorzugt 1, 2 oder 3 und r und s ergeben sich entsprechend der Stöchiometrie und Ladung des Metallions.

Besonders bevorzugt handelt es sich bei dem Anion in Formel I um Tetrafluoroborat, Hexafluorophosphat, Trifluormethansulfonat, Methansulfonat, Formiat, Acetat, Malonat, Succinat, Anion der Phthalsäure oder Trimellithsäure, Mandelat, Nitrat, Nitrit, Trifluoracetat, Sulfat, Hydrogensulfat, Methylsulfat, Ethylsulfat, Propylsulfat, Butylsulfat, Pentylsulfat, Hexylsulfat, Heptylsulfat, Octylsulfat, Phosphat, Dihydrogenphosphat, Hydrogenphosphat, Propionat, Tetrachloroaluminat, Al₂Cl₇-, Chlorozinkat, Chloroferrat, Bis-(trifluoromethylsulfonyl)imid, Bis(pentafluoroethylsulfonyl)imid, Tris(trifluoromethyl-sulfonyl)methid, Bis(pentafluoroethylsulfonyl)methid, p-Tolylsulfonat, Bis[salicylato(2-)]-borat, Tetracarbonylcobaltat, Dimethylenglykolmonomethylethersulfat, Octylsulfat, Oleat, Stearat, Acrylat, Methacrylat, Maleinat, Hydrogencitrat, Vinylphosphonat, Bis(pentafluoroethyl)phosphinat, Bis[oxalato(2-)]borat, Bis[1,2-benzoldiolato(2-)-O,O']borat, Dicyanamid, Tris(pentafluoroethyl)trifluorophosphat, Tris(heptafluoropropyl)trifluorophosphat, Tetracyanoborat oder Chlorocobaltat ist.

Ganz besonders bevorzugte Anionen A in Formel I sind Halogenide, insbesondere Chlorid, Alkylsulfonate, insbesondere C1 bis C4 Alkylsulfonate, besonders bevorzugt Methylsulfonat, ganz oder teilweise fluorierte Carboxylate, insbesondere Trifluoracetat, und Thiocyanat (Rhodanid).

### Allgemeines zu den Salzen der Formel I

Bei den Salzen der Formel I handelt es sich vorzugsweise um ionische Flüssigkeiten d. h. um Salze, welche bei Normaldruck (1 bar) einen Schmelzpunkt kleiner 200°C, insbesondere kleiner 100°C, vorzugsweise kleiner 75°C haben. Ganz besonders bevorzugt handelt es sich um ein bei Raumtemperatur (21 °C) und Normaldruck (1 bar) flüssige Salze.

Salze der Formel I haben insbesondere ein. Molgewicht kleiner 1000 g/ Mol, insbesondere kleiner 750 g/Mol und besonders bevorzugt kleiner 500 g/Mol.

Besonders bevorzugt Salze sind Imidazoliumsalze der Formel 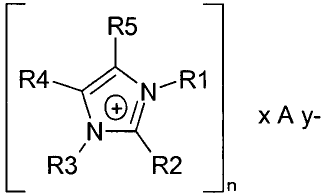 worin
R1 und R3 unabhängig voneinander für einen organischen Rest mit 1 bis 20 C-Atomen stehen
R2, R4, und R5 unabhängig voneinander für ein H-Atom oder für einen organischen Rest mit 1 bis 20 C-Atomen stehen,
A, n, x und y die obige Bedeutung haben, und
n, x und y stehen vorzugsweise jeweils für 1.

R1 und R3 stehen vorzugsweise unabhängig voneinander für eine organische Gruppe, die 1 bis 10 C-Atome enthält. Besonders bevorzugt handelt es sich um eine Kohlenwasserstoffgruppe, welche keine weiteren Heteroatome aufweist, z.B. um eine gesättigte oder ungesättigte aliphatische Gruppe, eine aromatische Gruppe oder eine Kohlenwasserstoffgruppe, welche sowohl aromatische als auch aliphatische Bestandteile aufweist. Ganz besonders bevorzugt handelt es sich um eine C1 bis C10 Alkylgruppe, eine C1 bis C10 Alkenylgruppe, z.B. eine Allylgruppe, eine Phenylgruppe, eine Benzylgruppe. Insbesondere handelt es sich um eine C1 bis C4 Alkylgruppe, z.B. eine Methylgruppe, Ethylgruppe, Propylgruppe, i-Propylgruppe oder n-Butylgruppe.

R2, R4 und R5 stehen vorzugsweise unabhängig voneinander für ein H-Atom oder für eine organische Gruppe, die 1 bis 10 C-Atome enthält. Besonders bevorzugt handelt es sich bei R2, R4 und R5 um ein H-Atom oder um eine Kohlenwasserstoffgruppe, welche keine weiteren Heteroatome aufweist, z.B. um eine aliphatische Gruppe, eine aromatische Gruppe oder eine Kohlenwasserstoffgruppe, welche sowohl aromatische als auch aliphatische Bestandteile aufweist. Ganz besonders bevorzugt handelt es sich um ein H-Atom oder eine C1 bis C10 Alkylgruppe, eine Phenylgruppe oder eine Benzylgruppe. Insbesondere handelt es sich um ein h.Atom oder eine C1 bis C4 Alkylgruppe, z.B. eine Methylgruppe, Ethylgruppe, Propylgruppe, i-Propylgruppe oder n-Butylgruppe.

A hat die oben genannte Bedeutung und steht insbeondere für ein Halogenide, insbesondere Chlorid, Alkylsulfonate, insbesondere C1 bis C4 Alkylsulfonate, besonders bevorzugt Methylsulfonat, ganz oder teilweise fluorierte Carboxylate, insbesondere Trifluoracetat, und Thiocyanat (Rhodanid).

### Zu dem Anion C in Formel II

Das Anion C in Formel II ist ein vom Anion A verschiedenes Carboxylat-Anion.

Vorzugsweise handelt es sich bei dem Carboxylat C um ein Carboxylat der Formel

R'-COO⁻

in dem
R'
- Wasserstoff;
- C₁- bis C₇-Alkyl;
- -OOC-(CH₂)ₙ- mit n gleich 0, 1 oder 2;
- R"OOC-(CH₂)ₙ- mit n gleich 0, 1 oder 2;
- -OOC-CH=CH-;
- R"OOC-CH=CH-;
- Ethenyl;
- 2-Propenyl;
- eine unsubstituierte oder durch eine bis fünf unabhängig voneinander ausgewählte Gruppen aus der Reihe C₁- bis C₆-Alkyl, Hydroxy, Carboxylat (-COO-), Carboxy (-COOH) und C₁- bis C₆-Alkyloxycarbonyl (-COOR# mit R# gleich C₁- bis C₆-Alkyl) substituierte Phenylgruppe
und
R" Wasserstoff oder C₁- bis C₆-Alkyl;
bedeuten.

Bei dem C₁- bis C₇-Alkyl-Rest handelt es sich beispielsweise um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl oder 3-Heptyl.

Bei der unsubstituierten oder durch eine bis fünf unabhängig voneinander ausgewählte Gruppen aus der Reihe C₁- bis C₆-Alkyl, Hydroxy, Carboxylat (-COO-), Carboxy (-COOH) und C₁- bis C₆-Alkyloxycarbonyl (-COOR² mit R² gleich C₁- bis C₆-Alkyl) substituierten Phenylgruppe handelt es sich beispielsweise um Phenyl, 2-Methylphenyl, 3-Methylphenyl, 4-Methylphenyl, 2-Hydroxyphenyl, 3-Hydroxyphenyl, 4-Hydroxyphenyl, 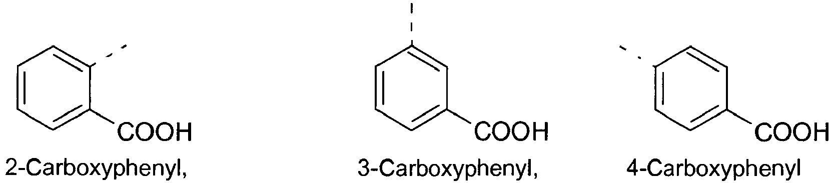 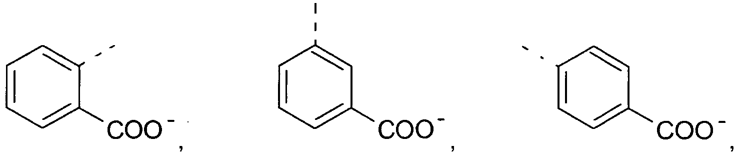 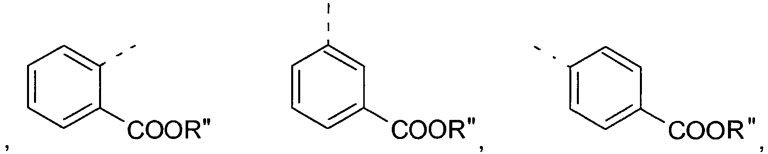 wobei R¹ für C₁- bis C₆-Alkyl steht.

Das beim erfindungsgemäßen Verfahren einzusetzende Carboxylat des entsprechenden heterocyclischen quartären Ammonium-Kations und/oder Guanidinium-Kations enthält bevorzugt als Carboxylat-Anion Formiat, Acetat, Propionat, Butyrat, Pentanoat (Valeriat), Hexanoat, Heptanoat, Octanoat, 2-Ethylhexanoat, Methyloxalat, Ethyloxalat, 1-Propyloxalat, 1-Butyloxalat, 1-Pentyloxalat, 1-Hexyloxalat, 1-Heptyloxalat, 1-Octyloxalat, 1-(2-Ethyl)hexyloxalat, Methylmalonat, Ethylmalonat, 1-Propylmalonat, 1-Butylmalonat, 1-Pentylmalonat, 1-Hexylmalonat, 1-Heptylmalonat, 1-Octylmalonat, 1-(2-Ethyl)hexylmalonat, Methylsuccinat, Ethylsuccinat, 1-Propylsuccinat, 1-Butylsuccinat, 1-Pentylsuccinat, 1-Hexylsuccinat, 1-Heptylsuccinat, 1-Octylsuccinat, 1-(2-Ethyl)hexylsuccinat, Methylmaleat, Ethylmaleat, 1-Propylmaleat, 1-Butylmaleat, 1-Pentylmaleat, 1-Hexylmaleat, 1-Heptylmaleat, 1-Octylmaleat, 1-(2-Ethyl)hexylmaleat, Methylfumarat, Ethylfumarat, 1-Propylfumarat, 1-Butylfumarat, 1-Pentylfumarat, 1-Hexytfumarat, 1-Heptylfumarat, 1-Octylfumarat, 1-(2-Ethyl)hexylfumarat, Acrylat, Methacrylat, Benzoat, 2-Methylbenzoat, 3-Methylbenzoat, 4-Methylbenzoat, 2-Hydroxybenzoat (Salicylat), 3-Hydroxybenzoat, 4-Hydroxybenzoat, o-Hydrogenphthalat, m-Hydrogenphthalat, p-Hydrogenphthalat, o-Phthalat, m-Phthalat, p-Phthalat, o-Methylphthalat, o-Ethylphthalat, o-(1-Propyl)phthalat, o-(1-Butyl)phthalat, o-(1-Pentyl)phthalat, o-(1-Hexyl)phthalat, o-(1-Heptyl)phthalat, o-(1-Octyl)phthalat, O-(1-(2-Ethyl)hexyl)-phthalat, m-Methylphthalat, m-Ethylphthalat, m-(1-Propyl)phthalat, m-(1-Butyl)phthalat, m-(1-Pentyl)phthalat, m-(1-Hexyl)phthalat, m-(1-Heptyl)phthalat, m-(1-Octyl)phthalat, m-(1-(2-Ethyl)hexyl)phthalat, p-Methylphthalat, p-Ethylphthalat, p-(1-Propyl)phthalat, p-(1-Butyl)phthalat, p-(1-Pentyl)phthalat, p-(1-Hexyl)phthalat, p-(1-Heptyl)phthalat, p-(1-Octyl)phthalat, p-(1-(2-Ethyl)hexyl)phthalat.

Besonders bevorzugt handelt es sich bei dem Carboxylat C um
R' Wasserstoff;
Methyl;
Ethyl; 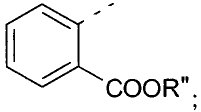 mit
R" C₁- bis C₄-Alkyl.

Ganz besonders bevorzugt ist das Carboxylat C ein Formiat, Acetat oder Propionat, insbesondere ein Acetat.

### Zum Verfahren

Für ionische Flüssigkeiten gibt es verschiedene Herstellungsverfahren.

Ionische Flüssigkeiten, insbesondere Imidazoliumsalze, können durch ein oder mehrstufige Umsetzung von Ausgangsverbindungen ausgewählt aus: α-Dicarbonylverbindungen, Aminoverbindungen, Carbonylverbindungen, Ammoniak und Carbonatverbindungen erhalten werden.

Als Herstellverfahren ist z.B. die Carbonatmethode bekannt, welche in WO 2005/021484 und beschrieben ist.

Bei der Carbonatmethode werden Imidazoliumsalze durch Umsetzung einer α-Dicarbonylverbindung , einer Carbonylverbindung (im allgemeinen Formaldehyd), einer Aminoverbindung und Ammoniak in einer ersten Stufe und einer anschließenden Umsetzung des Reaktionsprodukts in einer zweiten Stufe mit einem Carbonat (im allgemeinen Dimethylcarbonat) erhalten.

Ein weiteres Herstellungsverfahren für Imidazoliumsalze wurde von Arduengo et al. (WO 91/14678, Arduengo-Verfahren) beschrieben. Bei diesem einstufigen Verfahren erfolgt die Herstellung durch Umsetzung einer α-Dicarbonylverbindung, einer Carbonylverbindung (im allgemeinen Formaldehyd) und einer Aminoverbindung in Gegenwart einer Säure.

Bei den vorstehenden Verfahren werden Carboxylate erhalten. Falls ionische Flüssigkeiten, bzw. Imidazoliumsalze mit anderen Anionen (auch Carboxylate) gewünscht sind erfolgt im Anschluss ein Anionenaustausch.

Der Anionenaustausch erfolgt gemäß WO 2006/27070 durch Umsetzung mit einer Protonensäure des gewünschten Anions A.

Erfindungsgemäß erfolgt der Austausch mit dem Ammoniumsalz des Anions A oder mit der Protonensäure des Anion A in Gegenwart von Ammoniak

Ammoniak kann z.B. bereits vorab zur Protonensäure oder zur Verbindung mit dem auszutauschenden Anion A gegeben werden oder zum Gemisch der Protonensäure und der Verbindung mit dem auszutauschenden Anion C gegeben werden.

Die Umsetzung mit dem Ammonimsalz oder mit der Protonensäure in Gegenwart des Ammoniumsalzes erfolgt vorzugsweise bei 0°C bis 100°C, insbesondere 10 bis 60°C bei Normaldruck.

Während oder nach der Umsetzung kann das entstehendes Ammoniumsalz des Anions C (Ammoniumcarboxylat) bzw. Ammoniak und die entsprechende Protonensäure des Carboxylats aus dem Reaktionsgemisch entfernt werden, z.B. durch Destillation.

Geeignet sind übliche und dem Fachmann bekannte Destillationsverfahren. Vorteilhaft ist eine große Verdampferoberfläche im Verhältnis zum Flüssigkeitsvolumen. Geeignet sind daher insbesondere Destillationen mit Dünnschichtverdampfern, Fallfilmverdampfern oder Kurzwegdestillationen (Molekulardestillation).

Die Oberflächentemperatur beträgt vorzugsweise 110 bis 300°C, besonders bevorzugt 130 bis 280°C und ganz besonders bevorzugt 140°C bis 260°C.

Der Druck in dem Bereich zwischen Verdampferoberfläche und Kondensatoroberfläche beträgt vorzugsweise 0,0001 bis 10 mbar, bevorzugt 0,001 bis 5 mbar, besonders bevorzugt 0,05 bis 5 mbar.

Das Verfahren kann kontinuierlich oder diskontinuierlich durchgeführt werden.

Durch das erfindungsgemäße Verfahren kann in einfacher Weise ein vollständiger Anionenaustausch erfolgen. Der Austausch gelingt bereits in einem einzigen Reaktionsschritt, eine häufigere Wiederholung der Destillation zur Erreichung eines vollständigen Anionenaustausches ist nicht mehr notwendig.

### Beispiele

### Vergleichs-Beispiele V1 bis V3:

198 g (1mol) BMIM OAc (1-Butyl-3-Methyl-Imidazolium Acetat) werden in einem inertisierten Rundkolben mit Tropftrichter und Rückflusskühler vorgelegt. Es wird langsam unter Rühren die in der angehängten Tabelle angegebene Menge an Säure zugegeben, wobei die Temperatur unter 50 °C gehalten wird (bei der Zugabe wird eine exotherme Reaktion beobachtet; die Temperatur wird entweder durch entsprechend langsame Zugabe unter Luftkühlung oder durch Kühlen mit Wasser gehalten).

Nach Abkühlung auf Raumtemperatur wird der Großteil der flüchtigen Bestandteile bei 0,1 mbar Druck abgezogen, wobei die Innentemperatur bis auf 120°C gesteigert wird. Wenn aus der Mischung unter diesen Bedingungen keine Leichtsieder mehr ausgasen, wird abgekühlt und mit Stickstoff belüftet. Der Rückstand wird in die Vorlage der Kurzwegdestillation überführt und bei der unten angegebenen Verdampfertemperatur mit einer Geschwindigkeit von 100 ml/h zugefahren. Der Druck bei der Kurzwegdestillation wird auf 0,05 mbar eingestellt. Das Produkt wird als Sumpfablauf erhalten, so dass die Kurzwegdestillation hier als eine sehr effiziente Form der Leichsiederstrippung fungiert.

Die kondensierten Leichsieder bestehen aus Wasser (bei Zusatz eines wässrigen Reagenzes) und Essigsäure.

| Nr. | Säure | BMIM OAc : Säure | Verdampfer-Temp. | Produkt (Salz mit neuem Anion) | Ausbeute | Reinheit* |
|---|---|---|---|---|---|---|
| | | Mol:mol | °C | | % | |
| V1 | HCl; 35% in Wasser | 1 : 1,033 | 170 | BMIM Cl | 93,5 | Enthält 20 mol% HOAc nach erstem Durchgang Enthält 3 mol% HOAc nach zweitem Durchgang |
| V2 | CF3CO OH (Trifluoressigsäure) | 1 : 1,017 | 150 | BMIM TFA | 90,9 | Enthält 13 mol% HOAc nach erstem Durchgang Enthält 4 mol% HOAc nach zweitem Durchgang |
| V3 | CH3SO 3H (Methansulfonsäure) | 1 : 1,005 | 170 / 190 | BMIM CH3SO 3 | 92,3 | Enthält 9 mol% HOAc nach erstem Durchgang Keine HOAc nach dem zweiten Durchgang |

| | | | | | | |
|---|---|---|---|---|---|---|
| * Analyse durch H-NMR | | | | | | |

### Beispiele B1 bis B4 (erfindungsgemäß):

198 g (1mol) BMIM OAc werden in einem inertisierten Rundkolben mit Tropftrichter und Rückflusskühler vorgelegt. Es wird langsam unter Rühren die in der angehängten Tabelle angegebene Ammoniumsalz zugegeben, wobei die Temperatur unter 50°C gehalten wird (bei der Zugabe wird eine exotherme Reaktion beobachtet; die Temperatur wird entweder durch entsprechend langsame Zugabe unter Luftkühlung oder durch Kühlen mit Wasser gehalten).

Nach Abkühlung auf Raumtemperatur wird der Großteil der flüchtigen Bestandteile bei 3 mbar Druck abgezogen, wobei die Innentemperatur bis auf 130°C gesteigert wird. Wenn aus der Mischung unter diesen Bedingungen keine Leichtsieder mehr ausgasen, wird abgekühlt und mit Stickstoff belüftet. Der Rückstand wird in die Vorlage der Kurzwegdestillation überführt und bei der unten angegebenen Verdampfertemperatur mit einer Geschwindigkeit von 100 ml/h zugefahren. Der Druck bei der Kurzwegdestillation wird auf 0,05 mbar eingestellt. Das Produkt wird als Sumpfablauf erhalten, so dass die Kurzwegdestillation hier als eine sehr effiziente Form der Leichsiederstrippung fungiert.

| Nr. | Salz | BMIM OAc : Salz | Verdampfer-Temp. | Produkt Salz mit neuem Anion | Ausbeute | Reinheit* |
|---|---|---|---|---|---|---|
| | | Mol:mol | °C | | % | |
| B1 | NH4SCN | 1 : 1,011 | 160 | BMIM SCN | 86,9 | >95% (H-NMR) |
| B2 | NH4Cl | 1 : 1,013 | 200 | BMIM Cl | 85,6 | > 95% (H-NMR) |
| B3 | NH4 CF3COO | 1 : 1,01 | 155 | BMIM CF3CO O | 82,1 | >95% (H-NMR) |
| B4 | NH4 MeSO3 | 1: 1,02 | 190 | BMIM MeSO3 | 92,3 | >95% (H-NMR) |

| | | | | | | |
|---|---|---|---|---|---|---|
| * HNM-R | | | | | | |

### Beispiele B5 bis B7 (erfindungsgemäß):

198 g (1mol) BMIM OAc werden in einem inertisierten Rundkolben mit Tropftrichter und Rückflusskühler vorgelegt. Es wird langsam unter Rühren die in der angehängten Tabelle angegebene Menge an Säure, danach die angegebene Menge an Ammoniak-Lösung (25 Gew-% in Wasser) zugegeben, wobei die Temperatur jeweils unter 50°C gehalten wird (bei der Zugabe wird eine exotherme Reaktion beobachtet; die Temperatur wird entweder durch entsprechend langsame Zugabe unter Luftkühlung oder durch Kühlen mit Wasser gehalten).

Nach Abkühlung auf Raumtemperatur wird der Großteil der flüchtigen Bestandteile bei 3 mbar Druck abgezogen, wobei die Innentemperatur bis auf 130°C gesteigert wird. Wenn aus der Mischung unter diesen Bedingungen keine Leichtsieder mehr ausgasen, wird abgekühlt und mit Stickstoff belüftet. Der Rückstand wird in die Vorlage der Kurzwegdestillation überführt und bei der unten angegebenen Verdampfertemperatur mit einer Geschwindigkeit von 100 ml/h zugefahren. Der Druck bei der Kurzwegdestillation wird auf 0,05 mbar eingestellt. Das Produkt wird als Sumpfablauf erhalten, so dass die Kurzwegdestillation hier als eine sehr effiziente Form der Leichsiederstrippung fungiert.

| Nr. | Säure | BMIM OAc : Säure:NH3 | Verdampfer-Temp. | Produkt | Ausbeute | Reinheit* |
|---|---|---|---|---|---|---|
| | | Mol:mol:mol | °C | | % | |
| B5 | HCl (35% in Wasser) | 1 : 1,02: 1,01 | 200 | BMIM Cl | 91 | > 95% (H-NMR) |
| B6 | CF3COOH | 1 : 1,01: 1,02 | 155 | BMIM CF3COO | 87 | >95% (H-NMR) |
| B7 | MeSO3H | 1: 1,03:1,01 | 190 | BMIM MeSO3 | 95 | >95% (H-NMR) |

| | | | | | | |
|---|---|---|---|---|---|---|
| * HNM-R | | | | | | |

### Beispiele B8 und B9 (erfindungsgemäß):

170 g (1 mol) EMIM OAc (1-Ethyl-3-Methyl-Imidazolium Acetat) werden in einem inertisierten Rundkolben mit Tropftrichter und Rückflusskühler vorgelegt. Es wird langsam unter Rühren die in der angehängten Tabelle angegebene Ammoniumsalz zugegeben, wobei die Temperatur unter 50°C gehalten wird (bei der Zugabe wird eine exotherme Reaktion beobachtet; die Temperatur wird entweder durch entsprechend langsame Zugabe unter Luftkühlung oder durch Kühlen mit Wasser gehalten).

Nach Abkühlung auf Raumtemperatur wird der Großteil der flüchtigen Bestandteile bei 3 mbar Druck abgezogen, wobei die Innentemperatur bis auf 130 °C gesteigert wird. Wenn aus der Mischung unter diesen Bedingungen keine Leichtsieder mehr ausgasen, wird abgekühlt und mit Stickstoff belüftet. Der Rückstand wird in die Vorlage der Kurzwegdestillation überführt und bei der unten angegebenen Verdampfertemperatur mit einer Geschwindigkeit von 100 ml/h zugefahren. Der Druck bei der Kurzwegdestillation wird auf 0,05 mbar eingestellt. Das Produkt wird als Sumpfablauf erhalten, so dass die Kurzwegdestillation hier als eine sehr effiziente Form der Leichsiederstrippung fungiert.

| Nr. | Salz | EMIM OAc : Salz | Verdampfer-Temp. | Produkt | Ausbeute | Reinheit* |
|---|---|---|---|---|---|---|
| | | Mol:mol | °C | | % | |
| B8 | NH4SCN | 1 : 1,011 | 160 | EMIM SCN | 93 | >95% (H-NMR) |
| B9 | NH4 CF3COO | 1 : 1,01 | 155 | EMIM CF3CO O | 87 | >95% (H-NMR) |

| | | | | | | |
|---|---|---|---|---|---|---|
| * HNM-R | | | | | | |

### Beispiele B10 bis B12 (erfindungsgemäß):

184 g (1mol) EEIM OAc (1,3 Diethyl-ImidazoliumAcetat) werden in einem inertisierten Rundkolben mit Tropftrichter und Rückflusskühler vorgelegt. Es wird langsam unter Rühren die in der angehängten Tabelle angegebene Menge an Säure, danach die angegebene Menge an Ammoniak-Lösung (25 Gew-% in Wasser) zugegeben, wobei die Temperatur jeweils unter 50°C gehalten wird (bei der Zugabe wird eine exotherme Reaktion beobachtet; die Temperatur wird entweder durch entsprechend langsame Zugabe unter Luftkühlung oder durch Kühlen mit Wasser gehalten).

Nach Abkühlung auf Raumtemperatur wird der Großteil der flüchtigen Bestandteile bei 3 mbar Druck abgezogen, wobei die Innentemperatur bis auf 130°C gesteigert wird. Wenn aus der Mischung unter diesen Bedingungen keine Leichtsieder mehr ausgasen, wird abgekühlt und mit Stickstoff belüftet. Der Rückstand wird in die Vorlage der Kurzwegdestillation überführt und bei der unten angegebenen Verdampfertemperatur mit einer Geschwindigkeit von 100 ml/h zugefahren. Der Druck bei der Kurzwegdestillation wird auf 0,05 mbar eingestellt. Das Produkt wird als Sumpfablauf erhalten, so dass die Kurzwegdestillation hier als eine sehr effiziente Form der Leichsiederstrippung fungiert.

| Nr. | Säure | EEIM OAc : Säure:NH3 | Verdampfer-Temp. | Produkt | Ausbeute | Reinheit* |
|---|---|---|---|---|---|---|
| | | Mol:mol:mol | °C | | % | |
| B10 | HCl (35% in Wasser) | 1 : 1,01: 1,01 | 200 | EEIM Cl | 89 | > 95% (H-NMR) |
| B11 | CF3COOH | 1 : 1,02: 1,03 | 155 | EEIM CF3COO | 96 | >95% (H-NMR) |
| B12 | MeSO3H | 1: 1,03:1,01 | 190 | EEIM MeSO3 | 93 | >95% (H-NMR) |

| | | | | | | |
|---|---|---|---|---|---|---|
| * HNM-R | | | | | | |

## Patentansprüche

1. Verfahren zur Herstellung von Salzen der Formel I
(B⁺)ₙ × A^{y-}
wobei
B für ein Kation, welches ein heterocyclisches Ringsystem mit mindestens einem Stickstoffatom enthält,
A für ein Anion und
n für eine ganze Zahl von 1 bis 3 steht
x und y jeweils für eine ganze Zahl von 1 bis 3 stehen und das Produkt aus x und y gleich n ist
durch Umsetzung von Salzen der Formel II
(B⁺)ₙ × C^{y-}
worin B und n, x und y die obige Bedeutung haben und C für eine von A verschiedene Verbindung mit einer oder mehreren Carboxylatgruppen (kurz Carboxylate genannt) steht,
mit dem Ammoniumsalz des Anions A oder mit der Protonensäure des Anion A in Gegenwart von Ammoniak.

2. Verfahren gemäß r Anspruch 1 , **dadurch gekennzeichnet, dass** es sich um ein Imidazolium-Kation handelt.

3. Verfahren gemäß einem der Ansprüche 1 oder 2, **dadurch gekennzeichnet, dass** es sich dem Anion um Chlorid, Trifluoracetat, Methylsulfonat oder Rhodanid handelt.

4. Verfahren gemäß einem der Ansprüche 1 bis 3, **dadurch gekennzeichnet, dass** es sich bei dem Salz der Formel I um Imidazoliumsalze der Formel III 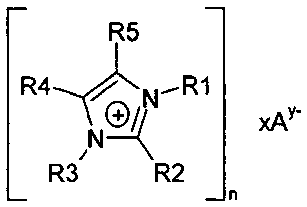 worin
A und n, x und y die obige Bedeutung haben,
R1 und R3 unabhängig voneinander für einen organischen Rest mit 1 bis 20 C-Atomen stehen und
R2, R4, und R5 unabhängig voneinander für ein H-Atom oder für einen organischen Rest mit 1 bis 20 C-Atomen stehen.

5. Verfahren gemäß einem der Ansprüche 1 bis 4, **dadurch gekennzeichnet, dass** es sich bei den Carboxylaten C in Formel II um Verbindungen mit 1 bis 20 C-Atomen und ein bis drei Carboxylatgruppen handelt.

6. Verfahren gemäß einem der Ansprüche 1 bis 5, **dadurch gekennzeichnet, dass** es sich bei den Carboxylaten C in Formel II um Anionen der C1 bis C10 Alkancarbonsäuren handelt.

7. Verfahren gemäß einem der Ansprüche 1 bis 6, **dadurch gekennzeichnet, dass** das Salz der Formel II mit dem Ammoniumsalz des Anions A umgesetzt wird.

8. Verfahren gemäß einem der Ansprüche 1 bis 7, **dadurch gekennzeichnet, dass** das Salz der Formel II mit der Protonensäure des Anion A in Gegenwart von Ammoniak umgesetzt wird.

9. Verfahren gemäß Anspruch 8, **dadurch gekennzeichnet, dass** die Umsetzung mit einer Lösung erfolgt, die Ammoniak und die Protonensäure des Anion A enthält.

10. Verfahren gemäß einem der Ansprüche 1 bis 9, **dadurch gekennzeichnet, dass** bei oder nach der Umsetzung das Ammoniumsalz des Anions C (Ammoniumcarboxylat) bzw. Ammoniak und die entsprechende Protonensäure des Carboxylats destillativ abgetrennt wird.

## Claims

1. A process for preparing salts of the formula I
(B⁺)ₙ x A^{y-}
where
B is a cation comprising a heterocyclic ring system having at least one nitrogen atom,
A is an anion and
n is an integer from 1 to 3,
x and y are each an integer from 1 to 3 and the product of x and y is equal to n,
by reacting salts of the formula II
(B⁺)ₙ x C^{y-}
where B and n, x and y are as defined above and C is a compound which has one or more carboxylate groups (referred to as carboxylate for short) and is different from A,
with the ammonium salt of the anion A or with the protic acid of the anion A in the presence of ammonia.

2. The process according to claim 1, wherein the cation is an imidazolium cation.

3. The process according to either of claims 1 and 2, wherein the anion is chloride, trifluoroacetate, methylsulfonate or thiocyanate.

4. The process according to any of claims 1 to 3, wherein the salt of the formula I is an imidazolium salt of the formula III 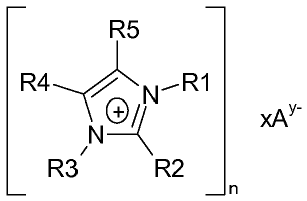 where
A and n, x and y are as defined above,
R1 and R3 are each, independently of one another, an organic radical having from 1 to 20 carbon atoms and
R2, R4 and R5 are each, independently of one another, an H atom or an organic radical having from 1 to 20 carbon atoms.

5. The process according to any of claims 1 to 4, wherein the carboxylate C in formula II is a compound having from 1 to 20 carbon atoms and from one to three carboxylate groups.

6. The process according to any of claims 1 to 5, wherein the carboxylate C in formula II is an anion of a C1 - C10-alkanecarboxylic acid.

7. The process according to any of claims 1 to 6, wherein the salt of the formula II is reacted with the ammonium salt of the anion A.

8. The process according to any of claims 1 to 7, wherein the salt of the formula II is reacted with the protic acid of the anion A in the presence of ammonia.

9. The process according to claim 8, wherein the reaction is carried out using a solution comprising ammonia and the protic acid of the anion A.

10. The process according to any of claims 1 to 9, wherein the ammonium salt of the anion C (ammonium carboxylate) or ammonia and the protic acid corresponding to the carboxylate is/are separated off by distillation during or after the reaction.

## Revendications

1. Procédé de fabrication de sels de formule I
(B⁺) ₙ x A^{y-}
dans laquelle
B représente un cation qui contient un système cyclique hétérocyclique contenant au moins un atome d'azote,
A représente un anion et
n représente un nombre entier de 1 à 3,
x et y représentent à chaque fois un nombre entier de 1 à 3 et le produit de x et y vaut n,
par réaction de sels de formule II
(B⁺) x C^{y-}
dans laquelle B et n, x et y ont la signification précédente et C représente un composé différent de A contenant un ou plusieurs groupes carboxylate (nommé carboxylates en abrégé),
avec le sel d'ammonium de l'anion A ou avec l'acide protonique de l'anion A en présence d'ammoniac.

2. Procédé selon la revendication 1, **caractérisé en ce qu'**il s'agit d'un cation d'imidazolium.

3. Procédé selon l'une quelconque des revendications 1 ou 2, **caractérisé en ce que** l'anion consiste en un chlorure, un trifluoroacétate, un méthylsulfonate ou un rhodanide.

4. Procédé selon l'une quelconque des revendications 1 à 3, **caractérisé en ce que** le sel de formule I consiste en des sels d'imidazolium de formule III 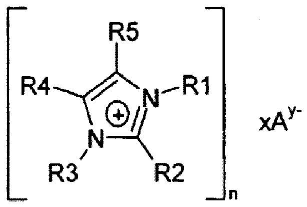 dans laquelle
A et n, x et y ont la signification précédente,
R1 et R3 représentent indépendamment l'un de l'autre un radical organique contenant 1 à 20 atomes C et
R2, R4 et R5 représentent indépendamment les uns des autres un atome H ou un radical organique contenant 1 à 20 atomes C.

5. Procédé selon l'une quelconque des revendications 1 à 4, **caractérisé en ce que** les carboxylates C dans la formule II sont des composés contenant 1 à 20 atomes C et un à trois groupes carboxylate.

6. Procédé selon l'une quelconque des revendications 1 à 5, **caractérisé en ce que** les carboxylates C dans la formule II sont des anions des acides alcanecarboxyliques en C1 à C10.

7. Procédé selon l'une quelconque des revendications 1 à 6, **caractérisé en ce que** le sel de formule II est mis en réaction avec le sel d'ammonium de l'anion A.

8. Procédé selon l'une quelconque des revendications 1 à 7, **caractérisé en ce que** le sel de formule II est mis en réaction avec l'acide protonique de l'anion A en présence d'ammoniac.

9. Procédé selon la revendication 8, **caractérisé en ce que** la réaction a lieu avec une solution contenant de l'ammoniac et l'acide protonique de l'anion A.

10. Procédé selon l'une quelconque des revendications 1 à 9, **caractérisé en ce que** lors de la réaction ou après la réaction, le sel d'ammonium de l'anion C (carboxylate d'ammonium) ou l'ammoniac et l'acide protonique correspondant du carboxylate sont séparés par distillation.
